# EUROPEAN PATENT APPLICATION

(11) **EP 1 350 799 A2**
(43) Date of publication of application: **08.10.2003**
(21) Application number: 03015364.7
(22) Date of filing: 01.12.1992
(51) Int. Cl.: C07K 16/28, C07K 16/46, C12N 15/13, C12N 15/85, C12N 5/10

(54) **Inhibitory Immunoglobulin Polypeptides to Human PDGF Beta Receptor**

(30) Priority: 02.12.1991 US 801795
(62) Divisional of application: 93900793.6
(71) Applicant: Millennium Pharmaceuticals, Inc., South San Francisco, California 94080 (US)
(72) Inventor: Ramakrishnan, Vanitha, Belmont, California 94002 (US); Escobedo, Maria A., San Francisco, California 94117 (US); Fretto, Larry J., Belmont, California 94002 (US)
(74) Representative: Thomson, Paul Anthony

(57) **Abstract**

An isolated immunoglobulin polypeptide or an antigen binding fragment thereof that specifically binds to an extracellular domain of the human PDGF-beta receptor.

SEQUENCE LISTING

(1) GENERAL INFORMATION:

(i) APPLICANT: Ramakrishnan, Vanitha
Escobedo, Maria A.
Fretto, Larry J.

(ii) TITLE OF INVENTION: Inhibitory Immunoglobulin
Polypeptides
to Human PDGF Beta Receptor

(iii) NUMBER OF SEQUENCES: 3

(iv) CORRESPONDENCE ADDRESS:
(A) ADDRESSEE: Townsend and Townsend
(B) STREET: One Market Plaza, Steuart Tower, Suite 2000
(C) CITY: San Francisco
(D) STATE: California
(E) COUNTRY: USA
(F) ZIP: 94105

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: PCT
(B) FILING DATE:
(C) CLASSIFICATION:

(vii) PRIOR APPLICATION DATA:
(A) APPLICATION NUMBER: US 07/801,795
(B) FILING DATE: 02-DEC-1991

(viii) ATTORNEY/AGENT INFORMATION:
(A) NAME: Smith, William M.
(B) REGISTRATION NUMBER: 30,223
(C) REFERENCE/DOCKET NUMBER: 12418-18

(ix) TELECOMMUNICATION INFORMATION:
(A) TELEPHONE: 415-326-2400
(B) TELEFAX: 415-326-2422

(2) INFORMATION FOR SEQ ID NO:1:

(i) SEQUENCE CHARACTERISTICS:
(A) LENGTH: 40 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

GTGTGAGGAA CGGGAAATTC ATCGAAGGAC ATCCCCCGAC
40

(2) INFORMATION FOR SEQ ID NO:2:

(i) SEQUENCE CHARACTERISTICS:
(A) LENGTH: 36 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:2:

GGAAGGTCGA TGTCTAGTTA ATCGAAGGAC ATCCCC
36

(2) INFORMATION FOR SEQ ID NO:3:

(i) SEQUENCE CHARACTERISTICS:
(A) LENGTH: 18 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:3:

Ser Val Leu Thr Leu Thr Asn Leu Thr Gly Leu Asp Thr Gly Glu
Tyr
1 5 10 15

Phe Cys

## Description

### FIELD OF THE INVENTION

The present invention relates generally to the production and use of immunoglobulin polypeptides that inhibit PDSF-mediated proliferation of cells displaying the human type beta platelet derived growth factor receptor.

### BACKGROUND OF THE INVENTION

Platelet derived growth factor (PDGF) is a potent proliferative agent in cells of mesenchymal origin (Antoniades, H.N. et al. (1979) Proc. Natl. Acad. Sci. USA 76: 1809-18137 Bowen-Pope, D.F. and Ross, R. (1982) J. Biol. Chem. 257: 5161-5171; Heldin, C-H. et al. (1983) J. Biol. Chem. 258: 10054-10059, all of which are incorporated herein by reference). PDGF (M.W. 30 KDa) is a disulphide-linked dimer consisting of 2 homologous chains termed A or B (Johnsson, A. et al. (1982) Biochim. Biophys. Res. Commun, 104: 66-74, which is incorporated herein by reference). The chains may combine with chains of the same or the other type, resulting in 3 isoforms AA, BB or AB (Heldin, C-H. et al. (1986) Nature 319: 511-514, which is incorporated herein by reference). The mitogen PDGF was first identified (Antoniades, H.N. (1979) Proc. Natl. Acad. Sci. USA 76: 1809-1813; Raines, E.W. and Ross, R. (1982) J. Biol. Chem. 257: 5154-5160, both of which are incorporated herein by reference) and purified from human platelets (Raines, op. cit.), though subsequent research has shown that several cell types including vascular endothelial cells, vascular smooth muscle cells, macrophages and even fibroblasts synthesise PDGF (Ross, R. et al. (1986) Cell 46; 155-169, which is incorporated herein by reference).

The cellular proliferation induced by all isoforms of PDGF is mediated by ligand binding to the PDGF receptor (Heldin, C-H. (19B3) op. cit., Ek, B. et al. (19B2) Nature 295: 419-420; Glenn, K. et al. (1982) J, Biol. Chem. 257: 5172-5176: Frackelton, A.R. et al. (1964) J. Biol. Chem. 259: 7909-7915; Williams, L.T. et al. (1984) J. Biol. Chem. 259: 5287-5294, all of which are incorporated herein by reference). The PDGF receptor (M.W. 180 KDa) belongs to the tyrosine kinase family and consists of two receptor subtypes, termed type A (or type alpha) (Matsui, T. et al. (1989) Science: 243: 800-804, and Claesson-Welsh, L. (1969) Proc._Natl. Acad. Sci. USA 86: 4917-4921, both of which are incorporated herein by reference) and type B (or type beta) (Yarden, Y. et al. (1986) Nature 323: 226-232, and Escobedo, J.A. et al. (1988) Science 240: 1532-1534, both of which are incorporated herein by reference).

High affinity binding of PDGF to the receptor is followed by receptor dimerisation (Dishayee, S. et al. (1989) J. Biol., chem. 264: 11699-11705, and Heldin, C-H. et al. (1989) J. Biol. Chem, 264: 8905-B912) and autophosphorylation (Frackelton, et al- op. cit.), and results in a complicated series of intracellular signalling events culminating in DNA synthesis. Mouse and human PDGF beta receptor and PDGF alpha receptor genes have been cloned (Matsui et al. op. cit.. Claesson-Welsh et al. op. cit., Yarden et al. op. cit., and Escobedo et al. op. cit.). When referring to PDGF receptors herein, type A and type alpha are used interchangeably, as are type B and type beta.

The two receptor isoforms may be distinguished by their markedly different ligand binding specificities. PDGF beta receptor binds only B-chain (isoforms BB and AB), while PDGF alpha receptor can bind all forms of PDGF (isoforms containing A and/or B chain (Matsui et al. op. cit., Claesson-Welsh et al op. cit., and seifert, R.A. et al. (1989) J. Biol. chem. 264: 8771-8778). The PDGF receptor shows a high degree of structural homology to the macrophage-colony stimulating factor receptor (Coussens, L. et al. (1986) Nature 320: 277-280) and the c-kit protooncogene product (Yarden, et al., op. cit.).

The PDGF receptors are characterised by an extracellular domain which may be demarcated into five Ig-like domains (Domains 1-5) based on their β-sheet rich structure. These Ig repeats of approximately 100 amino acids each have regularly spaced cysteine residues (except in the fourth repeat) . The receptor has a single transmembrane domain and a cytoplasmic tyrosine kinase domain (Williams, L. T. (1989) Science 243: 1564-1570, which is incorporated herein by reference).

PDGF plays an important role during normal physiological processes such as tissue repair and embryogenesis (Ross, R. et al. op, cit.). However, studies now implicate this potent mitogen in pathological proliferative disorders and in the development of certain carcinomas (ROBS, R. et al. op. cit.). Expression of PDGF A chain and PDGF beta receptor has been detected in human atherosclerotic plaques by In situ hybridisation (Wilcox, J.N. et al. (198B) J. Clin. Invest. 82: 1134-1143). Recently, Ferns et al. ((1991) Science 253: 1129-1132) have reported that a polyclonal antibody to PDGF significantly reduced the formation of intimal lesions in deendothelialised carotid arteries of athymic nude rats. PDGF has been implicated in the pathology of proliferative diseases in cells of mesenchymal origin (Nister, M. et al. (1984) Proc. Natl. Acad. Sci. USA 81: 926-930, and Nister, M. et al. (1987) cancer Res. 47: 4953-4961, both of which are incorporated herein by reference). Golden et al. have reported that PDGF A chain message was increased in areas of intimal hyperplasia in a baboon model for vascular grafts ((1990) J. Vasc. Surg. 11: 580-585). PDGF is also chemotactic for smooth muscle (Westermark, B. et al. (1990) Proc. Natl. Acad. Sci. USA 87: 128-132), and platelet PDGF may be the causative agent for the migration and proliferation of smooth muscle cells in the ballooned rat carotid artery, which results in significant stenosis.

The study of other growth factors and their receptors has been aided by the invention of antibodies against the receptors. For example, antibodies that recognize the epidermal growth factor receptor have proved to be powerful tools in evaluating the mechanism of receptor activation (Spaargaren, M, et al. (1991) J. Biol. Chem. 266; 1733-1739, which is incorporated herein by reference). Antibodies against receptors for interleukin-2 (IL-2) inhibit IL-2 internalisation, and thus inhibit the subsequent induction of proliferation of responsive cells (Duprez, V, et al, (1991) J. Biol. chem. 1497-1501, which is incorporated herein by reference). Similarly, a monoclonal antibody against the epidermal growth factor (EGF) receptor inhibits estrogen-stimulated growth of the human mammary adenocarcinoma cell line MCF-7 (Eppstein, D.A. (1989) J. cell. Physiol. 141: 420-430, which is incorporated herein by reference). Such antibodies may be of great therapeutic value in treating growth factor-mediated diseases.

Several groups have isolated antibodies against PDGF receptors, but these antibodies have limited Utility (see, for example, Kawahara, R.S. et al- (1987) Biochem. Biophys. Res. Commun. 147: 839-845, which is incorporated herein by reference). Additional monoclonal antibodies have been raised against the extracellular PDSF-binding domain of a PDGF receptor from porcine uterus (Ronnestrand, L. and Terracio, L. (19BB) J. Biol. Chem. 263: 10429-10435, which is incorporated herein by reference), but these antibodies did not inhibit binding of ¹²⁵I-labelled PDGF to human fibroblasts. Numerous antibodies against a PDGF receptor that did not inhibit PDGF activity have also been reported by Kanakaraj, P.S. et al. (1991) Biochemistry 30: 1761-1767; Claesson-Welsh, L. et al. (1989) J. Biol. Chem. 264; 1742-1747; Seifert, R. A. et al. (1989) J. Biol. Chem, 264: 8771-8778: Kumjian, D. A. et al. (1989) Proc. Natl. Acad. Sci. USA 86: 8232-8236; Bishayee, S. et al. (1988) Mol. Cell. Biol. 8: 3696-3702: Hart, C. E. et al. (1987) J. Biol. Chem. 262: 10780-107B5; Escobedo, J. A. et al. (1988) J. Biol. chem. 263: 1482-1487; Daniel, T. O. et al. (1987) J. Biol. Chem. 262: 977B-9784; Keating, M. T. and L. T. Williams (1987) J. Biol. Chem. 262: 7932-7937; Kazlauskas, A. and J. A. Copper (1990) EMBO J. 9: 3279-3286; all of which are incorporated herein by reference.

Thus, there exists a need for immunoglobulin and other agents capable of specifically inhibiting activation of the human receptor and/or proliferation of cells displaying the human type beta PDGF receptor, Such agents would be useful in mapping the different functional domains of the receptor, and in dissecting the role of PDGF and its receptors in normal and disease processes. Furthermore, such agents will have therapeutic value in the treatment of PDGF-mediated proliferative diseases, and also diseases involving PDGF-mediated chemotaxis and migration. Such diseases include:
a) restenosis, including coronary restenosis after angioplasty, atherectony, or other invasive methods of plaque removal, and renal or peripheral artery restenosis after the same procedures;
b) vascular proliferative phenomena and fibrosis associated with other forms of acute injury such as; pulmonary fibrosis associated with adult respiratory distress syndrome, renal fibrosis associated with nephritis, coronary stenosis associated with Kawasake's disease, and vascular narrowings associated with other arteritides such as Takayasha's disease;
c) prevention of narrowings in vein grafts;
d) prevention of narrowings due to accelerated smooth muscle cell migration and proliferation in transplanted organs;
e) other fibrotic processes, such as schleroderma, myofibrosis; and
f) inhibition of tumor cell proliferation which is mediated by PDGF.

The present invention fulfills these and other needs.

### SUMMARY OF THE INVENTION

The present invention provides immunoglobulin polypeptides that specifically bind to a human type beta platelet derived growth factor receptor (βPDGF-R), wherein binding of the inmunoglobulin polypeptide to the fifth Ig-like domain of a human βPDGF-R has one or more of the following effects; i) inhibition of PDGF BB binding to the receptor; ii) inhibition of PDGF-induced βPDGF-R phosphorylation; iii) inhibition of PDGF-induced dimerization of βPDGF-R; iv) inhibition of PDGF-induced mitogenesis of cells displaying human βPDGF-R; and v) PDGF-induced chemotaxis and migration of cells displaying βPDGF-R. A preferred embodiment of the invention is a monoclonal antibody, such as the monoclonal antibody 2A1E2, which is of the IgG₁ isotype.

Isolated nucleic acids having a sequence substantially identical to those coding for all or part of an immunoglobulin polypeptide having the described properties are also included in the invention. A cell line transfected, transformed, or infected with these nucleic acids is another embodiment of the invention, as is a method of producing the immunoglobulin polypeptide or fragments thereof by growing a cell line containing the claimed nucleic acids and harvesting the immunoglobulin polypeptides or fragments.

The immunaglobulin polypeptides of the invention have diagnostic as well as therapeutic uses. For example, a further aspect of the invention involves methods of treating a human having a PDGF-mediated disease involving proliferation of smooth muscle cells, comprising administering to the patient a therapeutically effective dose of an immunoglobulin polypeptide of the present invention.

### BRIEF DESCRIPTION OF THE FIGURES

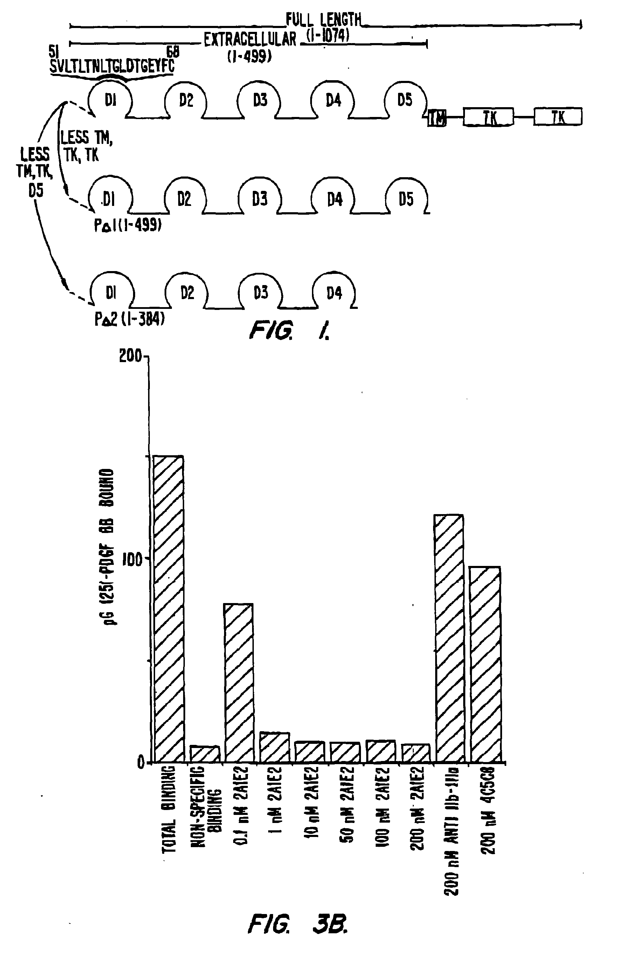

Fig 1. Recombinant Human Beta Receptor Extracellular Domain constructs. Deletion mutagenesis of the full length PDGF beta receptor was performed as described in Methods. PΔ1 refers to the 5 domain extracellular PDGF beta receptor (amino acids 1-499) which was made by deleting, from the PDGF type beta receptor cDNA, the codons for amino acid residues 500-1074 using the oligonucleotide GTG TGA GGA ACG GGA AAT TCA TCG AAG GAC ATC CCC CGAC (SEQ ID No:1). PΔ2 refers to the 4 domain extracellular PDGF receptor (aa 1-384) which was made by deleting the conds for amino acid residues 385-1074 from the cDNA, using the oligonucleotide GGA AGG TCGATG TCT ACT TAA TCG AAG GAC ATC CCC CGAC (SEQ ID NO:2). Putative Ig domains are indicated as follows: D1 (aa 1-91), D2 (aa 92-1B1), D3 (aa 182-282), D4 (aa 283-384) and D5 (aa 385-499), The peptide determinant of the polyclonal antisera 1-3-5 is indicated above D1.

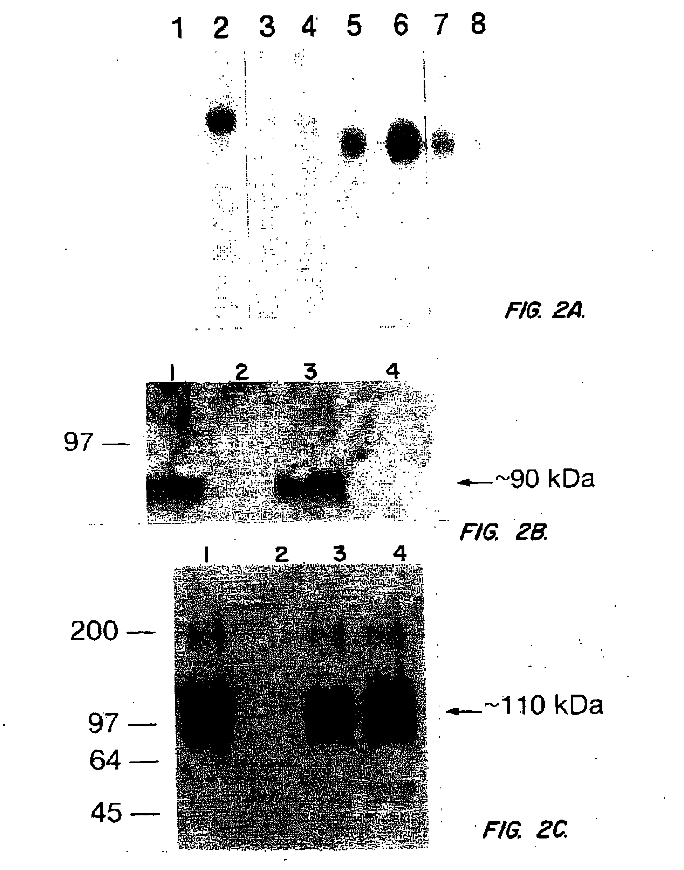

Fig 2. A. western Blot of Secreted 5 Domain Extracellular PDGF Beta Receptor pΔ1-5. Reduced (Lanes 1-3) and non-reduced (Lanes 4-6) secreted extracellular domain of PDGF beta receptor (PΔ1-5, 5 µg/lane) was electrophoresed, on 7% Laemmli gels, followed by Western transfer as described in Methods, The nitrocellulose was blocked in phosphate-buffered saline (PBS) containing 2% milk, cut into strips and incubated overnight at 4°C with 60 µg/ml of either MAb 2A1E2 (Lanes 1 and 4), another PDGF beta receptor monoclonal antibody (1C7D5) (Lanes 2 and 5), or a non-specific monoclonal antibody (Lanes 3 and 6). The nitrocellulose strips were washed with PBS containing 0.5% milk and 0.1% Tween 20, incubated with ¹²⁵I-labelled protein A for 2 hours at room temperature, and exposed to X-ray film. The arrow indicates the position of pΔ1-5.
   B. Immunoprecipitation of Secreted 4 Domain Extracellular PDGF Beta Receptor pΔ2-7. Secreted 4 domain extracellular PDGF beta receptor pΔ2-7 (2.6 µg) was incubated with either MAb 2A1E2 (Lane 2, 5 µg), 1C7D5 (Lane 3, 5 µg) or nonspecific MAb (Lane 4, 5 µg) in a final volume of 500 µl in I.P, buffer for 3 hours at 4°C. Protein A sepharose CL4B: Protein G sepharose CL4B (1:1) was added to each tube (60 µl of 50% slurry) and the incubation was continued for 2 hours at 4°C. The beads were spun down, washed 5X in I.P. buffer and electrophoresed on a 10% laemmli gel. The gel was transferred onto nitrocellulose and blocked in PBS containing 5% milk. The blot was incubated with a 1:100 dilution of a rabbit polyclonal anti-PDGF beta receptor Ab (1-3-5) in PBS containing 0.5% milk. After incubating overnight, the blot was washed, incubated with ¹²⁵I-Protein A, washed, and exposed to X-ray film. Lane 1 shows a standard of PΔ2-7 without immunoprecipation. The arrow indicates the position of pΔ2-7.
   C. Immunoprecipitation of Secreted Extracellular PDGF Beta Receptor by MAb 2AlE2. Extracellular human PDGF beta receptor (pΔ1-5, 5 µg) was immunoprecipitated with 5 µg of either nonspecific MAb (lane 2), MAb 2A1E2 (Lane 3), or 1C7D5 (Lane 4), as described for panel A. The samples were were processed and the blot was incubated with rabbit polyclonal anti-PDGF beta receptor 1-3-5 (1:100 dilution) and ¹²⁵I-protein A, as described in the legend for Panel B. Lane 1 contains 5 µg of standard pΔ1-5.

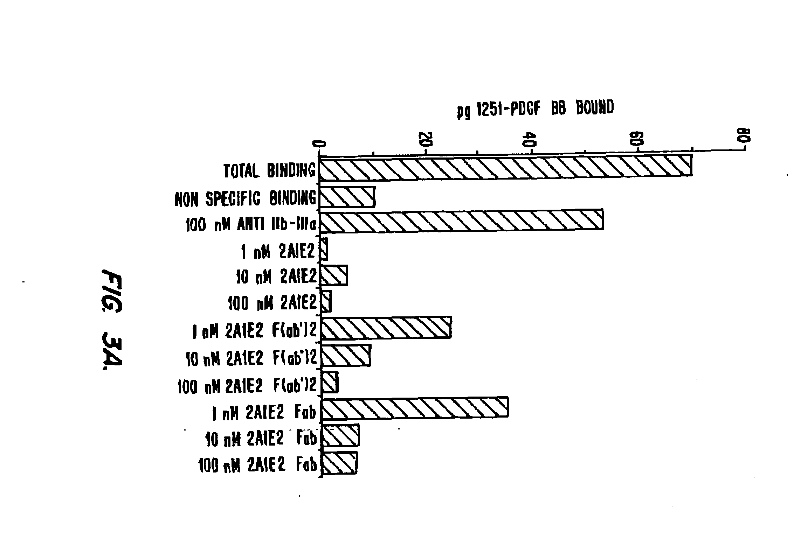

Fig 3, Inhibition of ¹²⁵I-PDGF BE Binding to HR5 Cells. A) HR5 cells were incubated with various concentrations of MAb 2A1E2 or control MAbs (200 nM anti-IIb/IIIa or 200 nM 4C5C8) as described in Methods. The cells were then incubated with ¹²⁵I-PDGF BB and the bound radioactivity was determined as described. Non-specific binding is defined as the amount of ¹²⁵I-PDGF BB bound in the presence of 100-fold excess unlabelled PDGF BE. B) HR5 cells were incubated with various concentrations of full-length MAb 2A1E2, MAb 2A1E2-F(ab') 2, or MAb 2A1E2-Fab, and 100 nM full-length anti-Ilb/IIIa as described in Methods. Total binding of ¹²⁵ I-PDGF BB to the cells in the presence and absence of MAbs and their derivatives was measured. The amount of ¹²⁵I-PDGF BB bound in the presence of 100 fold-excess unlabelled PDGF BB represents non-specific, binding.
Fig 4. Inhibition of Phosphorylation of HR5 cells by MAb 2A1E2. Confluent monolayers of HR5 cells in 6-well dishes were preincubated in duplicate with various MAbs, followed by incubation with ligand PDGF BB as described in Methods. Cells were solubilised, and the equivalent of one 6-well dish was electrophoresed on a 7% Laemmli gel and transferred onto nitrocellulose. The western blot was blocked and then incubated with antiphosphotyrosine MAb. The blot was then incubated with ¹²⁵I-Protein A and autoradiographed. Lanes 3-7 represent wells which were preincubated with either 0.13 nM MAb 2A1E2 (Lane 3), 1.3 nM MAb 2A1E2 (Lane 4), 13.3 nM MAb 2A1E2 (Lane 5), 0.13 µM MAb 2A1E2 (Lane 6) or 0.53 µM MAb 2A1E2 (Lane 7), followed by 100 ng/ml PDGF BB. Lane 2 shows the degree of phosphorylation in the presence of 100 ng/ml PDGF BE, when the cells were first preincubated with 0,53 µM of a non-specific MAb, and Lane 8 shows PDGF BB-induced phosphorylation when cells are preincubated with 0.53 µM of PDGF beta receptor MAb 4C5CB. The arrow indicates the position of the full length human PDGF beta receptor.

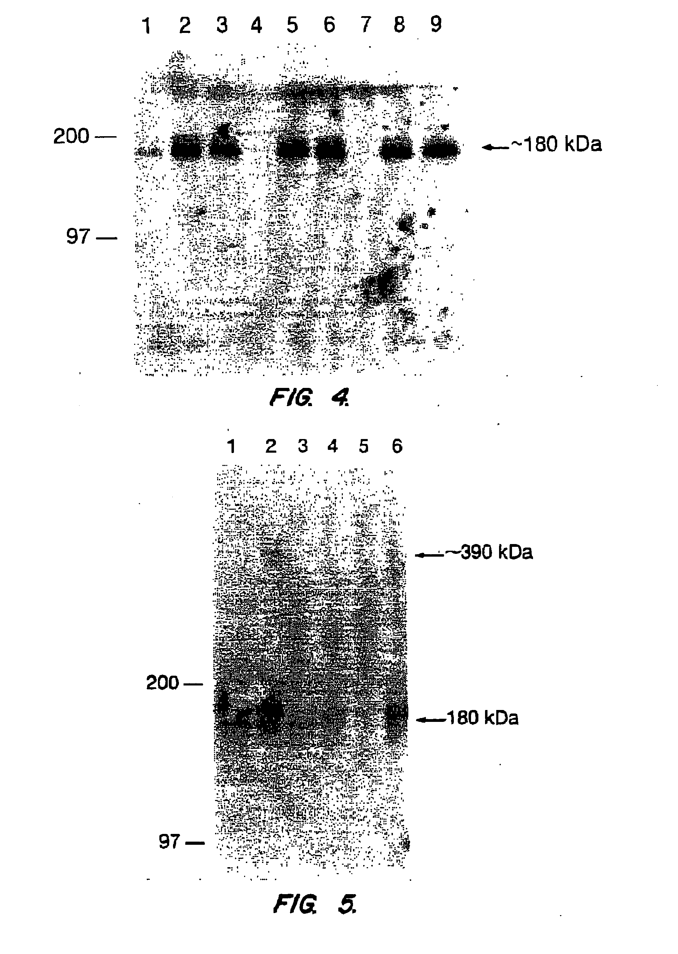

Fig 5. Inhibition of PDGF BB-Mediated Dimerisation of PDGF receptor by MAb 2A1E2. HR5 cells were incubated with either 13 nM MAb 2A1E2 (Lane 3), 0.13 µM MAb 2A1E2 (Lane 4), or 1.3 µM MAb 2A1E2 (Lane 5), followed by 100 ng/ml PDGF BB, and cross-linking was carried out as described in Methods. Lane 6 represents the effect of 0.1 µM anti-IIb/IIIa MAb on dimerisation. Lane 1 shows the relative amount of dimer in the absence of added crosslinker and lane 2 shows the amount of dimerized PDGF receptor in the absence of antibody.

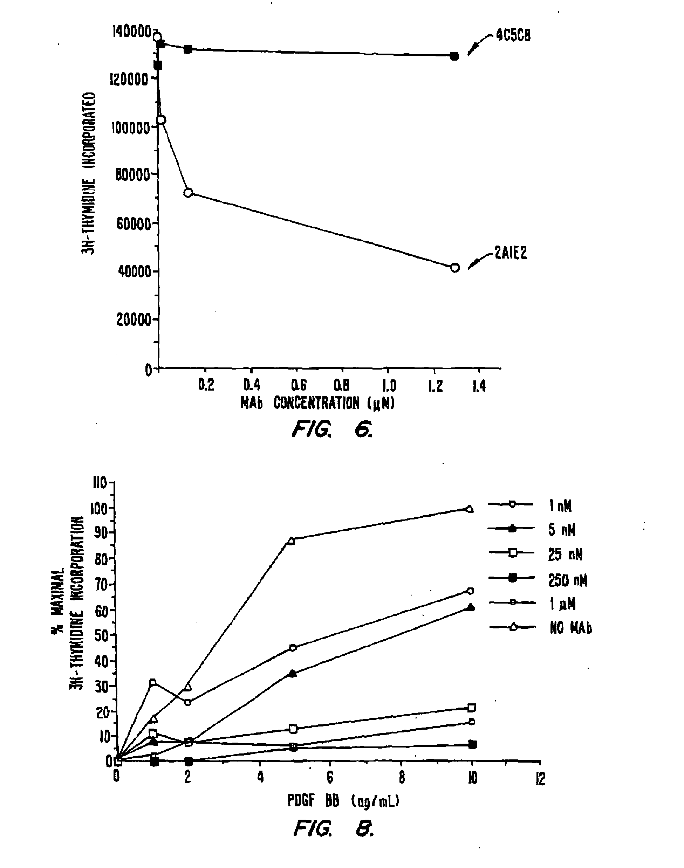

Fig 6. Inhibition of Mitogenesis in AG01523B cells by MAb 2A1E2. Cells were grown to confluence and incubated as described in Methods with various concentrations of either MAb 2A1E2 (open circles) or non-inhibitory control PDGF beta receptor MAb 4C5CB (solid squares) in the presence of 50 ng/ml of PDGF BB. ³H-thymidine incorporation was measured as described.

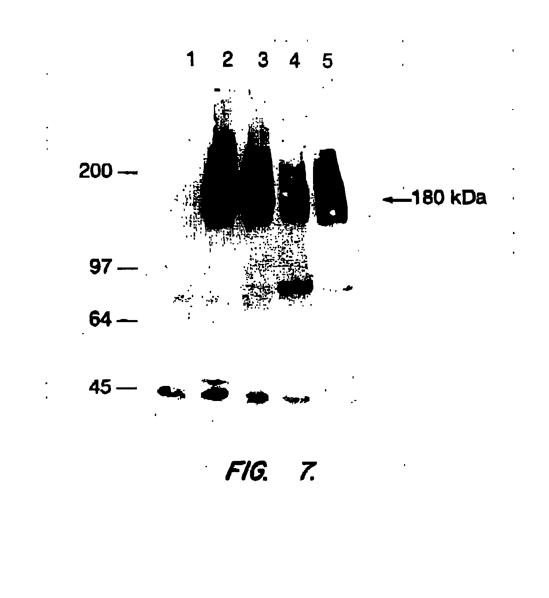

Fig 7. Inhibition of Phosphorylation by MAb 2A1E2 in Baboon Smooth Muscle Cells. Baboon smooth muscle cells were incubated with no PDGF BB (Lane 1), or with 100 ng/mL PDGF BB in the absence of MAb (Lane 2), or in the presence of 2 nM MAb 2A1E2 (Lane 3), 200 nM MAb 2A1 E2 (Lane 4) or 20 nM MAb 2A1E2 (Lane 5). Ligand induced phosphorylation was determined by Western analysis, as described in Methods.
Fig 8. Inhibition of Mitogenesis by MAb 2A1E2 in Baboon Smooth Muscle Cells. confluent baboon smooth muscle cells were incubated with 1 nM, 5 nM, 25 nM, 250 nM, or 1 µM MAb 2A1E2 in the presence of various concentrations of PDGF BB. ³H-Thymidine incorporation was measured as described in Methods. Data is expressed as a percent of maximal ³H-thymidine incorporated (approximately 30-50000 cpm) at saturating ligand concentration (10 ng/ml) in the absence of MAb. ³H-Thymidine incorporation in the absence of PDGF BB is 5-8000 cpm. The graph represents the average of 4 separate experiments.

### DESCRIPTION OF THE PREFERRED EMBODIMENTS

The present invention provides an immunoglobulin polypeptide that specifically binds to the human type beta PDGF receptor. The antibody is capable of inhibiting PDGF-induced mitogenesis of cells that display the human beta type PDGF receptor on the cell surface. The invention will be useful in diagnostic applications, and also for treating diseases involving PDGF-mediated proliferation, migration and chemotaxis of cells displaying the human type beta PDGF receptor.

### Definitions

### a) Proteins.

The terms "peptide", "polypeptide" or "protein" are used interchangeably herein. The term "substantial identity", when referring to polypeptides, indicates that the polypeptide or protein in question is at least about 30% identical to an entire naturally occurring protein or a portion thereof, usually at least about 70% identical, and preferably at least about 95% identical.

As used herein, the terms "isolated", "substantially pure" and "substantially homogenous" are used interchangeably and describe a protein that has been separated from components which naturally accompany it. Typically, a monomeric protein is substantially pure when at least about 60 to 75% of a sample exhibits a single polypeptide backbone. Minor variants or chemical modifications typically share the same polypeptide sequence. A substantially purified protein will typically comprise over about 85 to 90% of a protein sample, more usually about 95%, and preferably will be over about 99% pure. Protein. purity or homogeneity may be indicated by a number of means well known in the art, such as polyacrylamide gel electrophoresis of a protein sample, followed by visualizing a single polypeptide band on a polyacrylamide gel upon staining. For certain purposes high resolution will be needed and HPLC or a similar means for purification utilized.

A polypeptide is substantially free of naturally-associated components when it is separated from the native contaminants which accompany it in its natural state. Thus, a polypeptide which is chemically synthesized or synthesized in a cellular system different from the cell from which it naturally originates will be substantially free from its naturally-associated components.

Proteins may be purified to substantial homogeneity by standard techniques well known in the art, including selective precipitation with such substances as ammonium sulfate, column chromatography, immunopurification methods, and others. See, for instance, R. Scopes, Protein Purification; Principles and Practice, Springer-Verlag: New York (1982), which is incorporated herein by reference,

### b) Nucleic acids.

Nucleic acids, as used herein, may be DNA or RNA. When referring to nucleic acids, the term "substantial identity" indicates that the sequences of two nucleic acids, or designated portions thereof, when optimally aligned and compared, are identical, with appropriate nucleotide insertions or deletions, in at least about 80% of the nucleotides, usually at least about 90% to 95%, and more preferably at least about 98 to 99.5% of the nucleotides.

Alternatively, substantial nucleic acid sequence identity exists when a nucleic acid segment will hybridize under selective hybridization conditions, to a complement of another nucleic acid strand.

"Substantially complementary" similarly means that one nucleic acid is identical to, or hybridizes selectively to, another nucleic acid. Typically, selective hybridization will occur when there is at least about 55% identity over a stretch of at least 14-25 nucleotides, preferably at least about 65% identity, more preferably at least about 75%, and most preferably at least about 90% identity. See, M. Kanehisa Nucleic Acids Res. 12:203 (1984), which is incorporated herein by reference.

Stringent hybridization conditions will typically include salt concentrations of less than about 1 M, more usually less than about 500 mM and preferably less than about 200 mM. Temperature conditions will typically be greater than 22°C, more typically greater than about 30°C and preferably in excess of about 37°C. As other factors may dramatically affect the stringency of hybridization, including base composition and size of the complementary strands, presence of organic solvents and extent of base mismatching, the combination of parameters is more important than the absolute measure of any one alone.

"Isolated" or "substantially pure", when referring to nucleic acids, refer to those that have been purified away from other cellular components or other contaminants, e.g., other cellular nucleic acids or proteins, by standard techniques, including alkaline/SDS treatment, CsCl banding, column chromatography, and others well known in the art. see, F. Ausubel, et al., ed. Current Protocols in Molecular Biology. Greene Publishing and Wiley-Interscience, New York (1987), incorporated herein by reference.

A nucleic acid is "operably linked" when it is placed into a functional relationship with another nucleic acid sequence. For instance, a promoter or enhancer is operably linked to a coding sequence if it affects the transcription of the sequence. Generally, operably linked means that the nucleic acid sequences being linked are contiguous and, where necessary to join two protein coding regions, contiguous and in reading frame.

Techniques for nucleic acid manipulation, such as subcloning nucleic acid sequences encoding polypeptides into expression vectors, labelling probes, DNA hybridization, and so on are described generally, for example in Sambrook et al. (1989) Molecular Cloning; A Laboratory Manual (2nd ed.), vols. 1-3, Cold Spring Harbor Laboratory, or Ausubel et al., ed. (19B7) op. cit., both of which are incorporated herein by reference.

"Expression vectors", "cloning vectors", or "vectors" are often plasmids or other nucleic acid molecules that are able to replicate in a chosen host cell. Expression vectors may replicate autonomously, or they may replicate by being inserted into the genome of the host cell, by methods well known in the art. Vectors that replicate autonomously will have an origin of replication or autonomous replicating sequence (ABS) that is functional in the chosen host cell(s). Often, it is desirable for a vector to be usable in more than one host cell, e.g., in E. coli for cloning and construction, and in a mammalian cell for expression.

Mammalian cell lines are often used as host cells for the expression of polypeptides derived from eukaryotes. Propagation of mammalian cells in culture is per se well known. See, Tissue Culture. Academic Press, Kruse and Patterson, ed. (1973), incorporated herein by reference. Host cell lines may also include such organisms as bacteria (e.g., E. coli or B. subtilis), yeast, filamentous fungi, plant cells, or insect cells, among others.

"Transformation" refers to the introduction of vectors containing the nucleic acids of interest directly into host cells by well known methods. Transformation methods, which vary depending on the type of host cell, include electroporation; transfection employing calcium chloride, rubidium chloride calcium phosphate, DEAE-dextran, or other substances; microprojectile bombardment; lipofection; infection (where the vector is an infectious agent); and other methods. See generally, Sambrook et al., (1989) op. cit. and Ausubel et al. (ed.), (1987) op. cit. Reference to cells into which the nucleic acids described above have been introduced is meant to also include the progeny of such cells.

### c) Antibodies.

As used herein, "immunoglobulin polypeptide" refers to molecules which have specific immunoreactive activity. Antibodies are typically tetramers of immunoglobulin polypeptides. As used herein, the term "antibody" refers to a protein consisting of one or more polypeptides substantially encoded by immunoglobulin genes. Immunoglobulin genes include those coding for the light chains, which may be of the kappa or lambda types, and those coding for the heavy chains, Heavy chain types are alpha, gamma, delta, epsilon and mu. The carboxy terminal portions of immunoglobulin heavy and light chains are constant regions, while the amino terminal portions are encoded by the myriad immunoglobulin variable region genes. The variable regions of an immunoglobulin are the portions that provide antigen recognition specificity. In particular, the specificity resides in the complementarity determining regions (CDRs), also known as hypervariable regions, of the immunoglobulins. The immunoglobulins may exist in a variety of forms including, for example, Fv, Fab, and F(ab)₂, as well as in single chains (e.g., Huston, et al., Proc. Nat. Acad. Sci. U.S.A., 85:5879-5883 (1988) and Bird, et al., Science 242:423-426 (1988), which are incorporated herein by reference). (See, generally, Hood, et al., "Immunology", Benjamin, N.Y., 2nd ed. (1984), and Hunkapiller and Hood, Nature. 323:15-16 (1986), which are incorporated herein by reference). Single-chain antibodies, in which genes for a heavy chain and a light chain are combined into a single coding sequence, may also be used.

"Monoclonal antibodies" may be obtained by various techniques familiar to those skilled in the art. Briefly, spleen cells from an animal immunized with a desired antigen are immortalized, commonly by fusion with a myeloma cell (see, Kohler and Milstein, Eur. J. Immunol. 6:511-519 (1976)). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host.

Monospecific immunoglobulins may also be produced by recombinant techniques in prokaryotic or eukaryotic host cells. "chimeric" antibodies are encoded by immunoglobulin genes that have been genetically engineered so that the light and heavy chain genes are composed of immunoglobulin gene segments belonging to different species. For example, the variable (V) segments of the genes from a mouse monoclonal antibody may be joined to human constant (C) segments. Such a chimeric antibody is likely to be less antigenic to a human than antibodies with mouse constant regions as well as mouse variable regions.

As used herein, the term chimeric antibody also refers to an antibody that includes an immunoglobulin that has a human-like framework and in which any constant region present has at least about 85-90%, and preferably about 95% polypeptide sequence identity to a human immunoglobulin constant region, a so-called "humanized" immunoglobulin (see, for example, PCT Publication WO 90/07861, which is incorporated herein by reference) . Hence, all parts of such a "humanized" inmunoglobulin, except possibly the complementarity determining regions (CDR's), are substantially identical to corresponding parts of one or more native human immunoglobulin sequences.

The term "framework region", as used herein, refers to those portions of immunoglobulin light and heavy chain variable regions that are relatively conserved (i.e., other than the CDR's) among different immunoglobulins in a single species, as defined by Kabat, et al., (1987): Sequences of Proteins of Immunoglogic Interest, 4th Ed., US Dept. Health and Human Services, which is incorporated herein by reference). As used herein, a "human-like framework region" is a framework region that in each existing chain comprises at least about 70 or more amino acid residues; typically 75 to 85 or more residues, identical to those in a human immunoglobulin.

Human constant region DNA sequences can be isolated in accordance with well known procedures from a variety of human cells, but preferably from immortalized B-cells, The variable regions or CDRs for producing the chimeric immunoglobulins of the present invention will be similarly derived from monoclonal antibodies capable of binding to the human type beta PDGF receptor, and will be produced in any convenient mammalian source, including, mice, rats, rabbits, or other vertebrate capable of producing antibodies by well known methods.

Suitable source cells for the DNA sequences and host cells for immunoglobulin expression and secretion can be obtained from a number of sources, such as the American Type culture Collection ("Catalogue of Cell Lines and Hybridomas," Fifth edition (1985) Rockville, Maryland, U.S.A., which is incorporated herein by reference).

In addition to the chimeric and "humanized" immunoglobulins specifically described herein, other substantially identical modified immunoglobulins can be readily designed and manufactured utilizing various recombinant DNA techniques well known to those skilled in the art. In general, modifications of the genes may be readily accomplished by a variety of well-known techniques, such as site-directed mutageneais (see, Gillman and Smith, Gene B:B1-97 (1979) and S. Roberts et al., Nature 328:731-734 (1997), both of which are incorporated herein by reference).

Alternatively, polypeptide fragments comprising only a portion of the primary immunoglobulin structure may be produced. For example, it may be desirable to produce immunoglobulin polypeptide fragments that **possess** one or more immunoglobulin activities in addition to, or other than, antigen recognition (e.g., complement fixation).

Immunoglobulin genes, in whole or in part, may also be combined with functional regions from other genes (e.g., enzymes), or with other molecules such as toxins or labels to produce fusion proteins (e.g., "immunotoxins") having novel properties. In these cases of gene fusion, the two components are present within the same polypeptide chain. Alternatively, the immunoglobulin or fragment thereof may be chemically bonded to the toxin or label by any of a variety of. well-known chemical procedures. For example, when the label or cytotoxic agent is a protein and the second component is an intact immunoglobulin, the linkage may be by way of heterobifunctional cross-linkers, e.g., SPDP, carbodiimide, glutaraldehyde, or the like.

Suitable labels include, for example, radionuclides, enzymes, substrates, cofactors, inhibitors, fluorescers, chemiluminescers, magnetic particles. See, for examples of patents teaching the use of such labels, U.S. Patent Nos. **3,817,837;** 3,850,752; 3,939,350; 3,996,345; 4,277,437; 4,275,149; and 4,366,241, all of which are incorporated by reference.

Immunotoxins, including single chain molecules, may also be produced by recombinant means. Production of various immunotoxins is well-known with the art, and methods can be found, for example in "Monoclonal Antibody-Toxin Conjugates: Aiming the Magic Bullet." Thorpe et al, Monoclonal. Antibodies in Clinical Medicine, Academic Press, pp. 168-190 (1982); E. Vitetta, Science (1997) 238;1098-1104; and G. Winter and C. Milstein, Nature (1991) 349:293-299; all incorporated herein by reference.

A variety of cytotoxic agents are suitable for use in immunotoxins. cytotoxic agents can include radionuclides, such as Iodine-131, Yttrium-90, Rhenium-188, and Bismuth-212; a number of chemotherapeutic drugs, such as vindesine, methotrexate, adriamycin, and cisplatinum; and cytotoxic proteins such as ribosomal inhibiting proteins like pokeweed antiviral protein, Pseudomonas exotoxin A, ricin, diphtheria toxin, ricin A chain, etc., or an agent active at the cell surface, such as the phospholipase enzymes (e.g., phospholipase C). (See. generally, "Chimeric Toxins," Olsnes and Pihl, Pharmac. Ther., 15:355-381 (1981), and "Monoclonal Antibodies for Cancer Detection and Therapy," eds. Baldwin and Byers, pp. 159-179, 224-266, Academic Press (1985), both of which are incorporated herein by reference).

### Description of the invention

The immunoglobulin polypeptides of the present invention will find use in therapeutics as well as in diagnostics and other applications. Various techniques useful in these arts are discussed, for example, in Harlow and Lane, Antibodies; A Laboratory Manual, Cold Spring Harbor, New York (1986) (incorporated herein by reference for all purposes), including: immunization of animals to produce immunoglobulins; production of monoclonal antibodies; labeling immunoglobulins for use as probes; immunoaffinity purification; and immunoassays.

An example of an immunoglobulin polypeptide of the present invention is the monoclonal antibody 2A1E2, described below, which binds specifically to the type beta human PDGF receptor. Monoclonal antibody 2A1E2, which is of the IgG₁ isotype, was deposited with the American Type Culture Collection, 12301 Park Lawn Drive, Rockville, Maryland 20231 (ATCC no. HB10938), prior to the filing date of this application.

The anti-PDGF receptor immunoglobulin polypeptides of the present invention may be prepared by immunizing an animal with a purified or partially purified extracellular domain of human beta-type PDGF receptor. The animals immunized can be any one of a variety of species which are capable of immunologically recognizing epitopes characteristic of the human type beta PDGF receptor extracellular domain, such as murine, lagomorph, equine, etc.

Monoclonal antibodies of the invention may be prepared by immortalizing nucleic acid sequences which encode immunoglobulin polypeptides or portions thereof that bind specifically to antigenic determinants characteristic of the extracellular domain of the human type beta PDGF receptor. The immortalization process can be carried out by hybridoma fusion techniques, by viral transformation of antibody-producing lymphocytes, recombinant DNA techniques, or by techniques that combine cell fusion, viral transformation and/or recombinant DNA methodologies.

According to one aspect of the invention, cells producing human anti-EDGY receptor monoclonal antibodies are immortalized using, e.g., Epstein-Barr virus. (EBV) transformation techniques. For example, B lymphocytes derived from peripheral blood, bone marrow, lymph nodes, tonsils, etc. of patients, preferably those immunized with the PDGF receptor or portions thereof, are immortalized using EBV according to methods such as those described in U.S. Patent No. 4,464,465, and Chan et al, J. Immunol. 136:106 (1986), which are incorporated herein by reference.

Human anti-PDGF receptor monoclonal antibodies can also be prepared by a variety of other ways, e.g., using a combination of EBV or other viral transformation and hybridoma fusion techniques. For instance, the hybridomas can be created by fusing stimulated B cells, obtained from a individual immunized with the PDGF receptor or a portion thereof, with a mouse/human heterohybrid fusion partner, a variety of which have been described. See, e.g., U.S. Pat. No. 4,624,921 and James and Bell, J. Immunol. Meths. 100:5-40 (1987), which are incorporated herein by reference, A mouse/human fusion partner can be constructed by fusing human lymphocytes stimulated or transformed by EBV with readily available mouse myeloma lines such as NS-1 or P3NS-1, in the presence of, e.g., polyethylene glycol, 4 The hybrid should be suitably drug-marked, which can be accomplished by cultivating the hybrid in increasing concentrations of the desired drug, such as 6-thioguanine, ouabain, or neomycin.

The hybridomas or lymphoblastoid cells which secrete antibody of interest can be identified by screening culture supernatants for antibody that binds to the type beta PDGF receptor. More preferably, a screening assay may be employed to detect those antibodies which inhibit, for example, PDGF-mediated mitogenesis. Cells which possess the desired activity are cloned and subcloned. in accordance with conventional techniques and monitored until stable, immortalized lines producing the anti-PDGF receptor monoclonal antibody of interest are' identified. By monoclonal antibody is meant an antibody produced by a clonal, immortalized cell line separate from cells producing antibodies with a different antigen binding specificity. Thus, such monoclonal antibodies are produced isolated from other monoclonal antibodies and, accordingly, in substantially pure form (relative to other antibodies) and at a concentration generally greater than normally occurring in sera from the animal species which serves as a B cell source.

Alternatively, one can isolate DNA sequences which encode a human anti-FDGF receptor immunoglobulin polypeptide or portions thereof that specifically bind to the extracellular domain of the EDGY receptor by screening a DNA library from human B cells according to a general protocol as outlined by Huse et al., Science 246:1275-1281 (1989), incorporated herein by reference, and then cloning and amplifying the sequences which encode the anti-PDGF receptor antibodies (or binding fragment) of the desired Specificity.

The immunoglobulins may then be produced by introducing an expression vector containing the appropriate immunoglobulin gene, or portion thereof, into an appropriate host cell. The host cell line is then maintained under conditions suitable for high level expression of the immunoglobulin nucleotide sequences, and, as desired, the collection and purification of the light chains, heavy chains, light/heavy chain dimers or intact antibodies, binding fragments or other immunoglobulin forms may follow.

Suitable host cells include microorganisms, but mammalian or insect tissue cell culture may be preferable for producing the monoclonal antibody of the present invention (see, E. winnacker, "From Genes to Clones," VCH Publishers, N.Y., N.Y. (1987), which is incorporated herein by reference). A number of suitable host cell lines capable of secreting intact inmunoglobulins have been developed in the art, and include the Chinese hamster ovary (CHO) cell line, but preferably transformed B-cells or hybridomas will be used.

Once expressed, the whole antibodies, their dimers, individual light and heavy chains, or other immunoglobulin forms of the present invention can be purified according to standard procedures of the art, including ammonium sulfate precipitation, affinity columns, column chromatography, gel electrophoresis and the like (see, generally. R. Scopes, Protein Purification. Springer-Verlag, N.Y. (1982), incorporated herein by reference). Substantially pure immunoglobulins of at least about 90 to 95t homogeneity are preferred, and those of 98 to 99% or greater homogeneity most preferred, for pharmaceutical uses. Once purified, partially or to homogeneity as desired, the polypeptides may then be used therapeutically (including extracorporeally) or in developing and performing assay procedures, immunofluorescent stainings, and the like. (See, generally, Immunological Methods, Vols. I and II, Lefkovits and Pernis, eds., Academic Press, New York, N.Y. (1979 and 1981), which are incorporated herein by reference).

The immunoglobulin polypeptides produced according to the present invention may be of the IgG, IgM, IgA or IgD isotype, and may further be any of the appropriate subclasses thereof, such as, e.g., IgG₁, IgG₂, IgG₃, or IgG₄. Using recombinant DNA techniques, "class-switching" of the isolated immunoglobulin polypeptides may be readily accomplished. In this method genes encoding the constant regions which determine the isotype of the immunoglobulin molecule of interest are replaced by genes encoding a desired isotype or subclass, as generally described in European patent publication EP 314,161, incorporated herein by reference. Class-switched **immunoglobulins** may also be isolated by selecting cells which have undergone spontaneous switching using selection methods known in the art.

The administration to humans of immonoglobulin polypeptides which are substantially non-human may elicit anti-antibody responses. Thus, it may be desirable to prepare anti-PDGF receptor immunoglobulin polypeptides of the present invention which are substantially human. By "substantially human" is meant an antibody or binding fragment thereof comprised of amino acid sequences which are at least about 50% human in origin, at least about 70 to 80% more preferred, and about 95-99% or more human most preferred, particularly for repeated administrations over a prolonged period as may be necessary to treat established PDGF-mediated cell proliferation disorders. As used herein, human antibody is meant to include antibodies of entirely human origin as well as those which are substantially human, unless the context indicates otherwise.

As the generation of human anti-PDGF receptor monoclonal antibodies may be difficult with conventional immortalization techniques, it may be desirable to first make non-human antibodies and then transfer via recombinant DHA techniques the antigen binding regions of the non-human antibodies, e.g., the Fab, complementarity determining regions (CDRS) or hypervariable regions, to human constant regions (Fc) or framework regions as appropriate to produce substantially human molecules, such methods are generally known in the art and are described in, for example, U.S. 4,816,397, PCT publication WO 90/07861, and EP publications 173494 and 239400, wherein each is incorporated herein by reference.

The resulting chimeric antibodies or chimeric inmunoglobulin polypeptides that specifically bind to the human type beta PDGF receptor and thus inhibit binding of PDGF to the receptor are also within the scope of the present invention. A typical therapeutic chimeric antibody would be a hybrid protein consisting of the variable (V) or antigen-binding domain from mouse immunoglobulin specific for a human PDGF type beta receptor antigenic determinant, and the constant (C) or effector domain from a human immunoglobulin, although domains from other mammalian species may be used for both variable and constant domains. As used herein, the term ''chimeric antibody" also refers to antibodies coded for by immunoglobulin genes in which only the complementarity determining regions (CDR'S) are transferred from the trimunoglobulin that specifically recognizes the antigenic determinants, the remainder of the immunoglobulin gene being derived from a human (or other mammalian, as desired) imnunoglobulin gene. This type of chimeric antibody is referred to as a "humanized" (in the case of a human immunoglobulin gene being used) antibody

The hypervariable regions of the variable domains of the anti-PDSF receptor immunoglobulin polypeptides comprise a related aspect of the invention. The hypervariable regions, or CDRs, in conjunction with the framework regions (those portions of immunoglobulin light and heavy chain variable regions that are relatively conserved among different immunoglobulins in a single species), enable the anti-PDGF receptor immunoglobulin polypeptides to recognize and thus bind to the human type beta PDGF receptor. The hypervariable regions can be cloned and sequenced. Once identified, these regions that confer specific recognition of the PDGF receptor can then be cloned into a vector for expression in a host as part of another immunoglobulin molecule or as a fusion protein, e.g., a carrier molecule which functions to enhance immunogenicity of the cloned idiotope.

The anti-PDGF receptor immunoglobulin polypeptides of the invention will generally be used intact, or as inmunogenic fragments, such as Fᵥ, Fab, or F(ab')₂ fragments. The fragments may be obtained from antibodies by conventional techniques, such as by proteolytic digestion of the antibody using, e.g., pepsin or papain, or by recombinant DNA techniques in which a gene or portion thereof encoding the desired fragment is cloned or synthesized, and expressed in a variety of hosts.

Those skilled in the art will realize that "anti-idiotypic" antibodies can be produced by using a specific immunoglobulin as an immunogen in accordance with standard techniques. For example, infection or immunization with a PDSF receptor polypeptide, or fragment thereof, induces a neutralizing immunoglobulin, which has on its Fab variable region combining site an image of the PDSF receptor polypeptide that is unique to that particular imnunoglobulin, i.e., an idiotype Immunization with such an anti-PDGF-R immunoglobulin induces an anti-idiotype antibody, which has a conformation at its combining site that mimics the structure of the original PDGF-R antigen. These anti-idiotype antibodies may therefore be used instead of the PDGF-R antigen to treat PDGF-mediated diseases (see, for example, Nisonoff (1991) J. Immunol. 147:2429-2438, which is incorporated herein by reference).

The anti-PDGF receptor immunoglobulin polypeptides of the invention find utility in therapeutic and diagnostic methods and compositions. For therapeutic uses, anti-PDGF receptor immunoolobulin polypeptides are used as a soluble ligand for human type beta PDGF receptor, masking the receptor or otherwise inhibiting PDGF molecules from binding to the receptor, and thereby inhibiting the undesired cell migration and proliferation.

For pharmaceutical compositions, the anti-PDGF receptor immunoglobulin polypeptides of the invention as described herein are administered to an individual having a PDGF-mediated cellular proliferation disorder. In therapeutic applications, compositions are administered to a patient in an amount sufficient to effectively block cell receptors, and thereby cure or at least partially arrest the cellular proliferation and its symptoms and/or complications. An amount adequate to accomplish this is defined as "therapeutically effective dose." Amounts effective for this use will depend on, e.g., the nature of the anti-PDGF receptor immunoglobulin polypeptide composition, the manner of administration, the stage and severity of the disease being treated, the weight and general state of health of the patient, and the judgment of the prescribing physician, but will generally range from about 0.01 mg/kg to about 100.0 mg/kg of antibody per day, with dosages of from about 0.1 mg/kg to about 10.0 mg/kg of antibody per day being more commonly used. It must be kept in mind that the anti-PDGF receptor immunoglobulin polypeptide and peptide compositions derived therefrom may be employed in serious disease states, that is, life-threatening or potentially life threatening situations. In such cases, it is possible and may be felt desirable by the treating physician to administer substantial excesses of these compositions. Thus, human anti-FDGF receptor monoclonal antibodies or substantially human anti-PDGF receptor nonoclonal antibodies of the invention are most preferred under these circumstances.

Single or multiple administrations of the compositions can be carried out with dose levels and pattern being selected by the treating physician. In any event, the pharmaceutical formulations should provide a quantity of anti-PDSF receptor immunoglobulin polypeptide of the invention sufficient to effectively treat the patient. Administration should begin at the first indication of undesirable cellular proliferation or shortly after diagnosis, and continue until symptoms are substantially abated and for a period thereafter. In well established cases of disease, loading doses followed by maintenance doses will be required.

The pharmaceutical compositions for therapeutic treatment are intended for parenteral, topical, oral or local administration. Preferably, the pharmaceutical compositions are administered parenterally, e.g., intravenously, subcutaneously, intradermally, or intramuscularly. Thus, the invention provides compositions for parenteral administration which comprise a solution of the anti-PDGF receptor immunoglobulin polypeptide dissolved or suspended in an acceptable carrier, preferably an aqueous carrier. A variety of aqueous carriers may be used, e.g., water, buffered water, 0.4% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of anti-PDGF receptor immunoglobulin polypeptides of the invention in the pharmaceutical formulations can vary widely, i.e., from less than about 1%, usually at or at least about 10-15% to as much as 50% or more by weight, and will be selected primarily by fluid volumes, viscosities, etc., in accordance with the particular mode of administration selected.

Thus, a typical pharmaceutical composition for intravenous infusion could be made up to contain 250 ml of sterile Ringer's solution, and 100 mg of anti-PDGF receptor imnunoglobulin polypeptide. Actual methods for preparing parenterally administrable compounds will be known or apparent to those skilled in the art and are described in more detail in for example, Remington's Pharmaceutical Science, 17th ed., Mack Publishing Company, Easton, PA (1985), which is incorporated herein by reference,

The anti-PDGF receptor immunoglobulin polypeptides and fragments thereof can also be administered via liposomes. The anti-PDSF receptor immunoglobulin polypeptides can serve to target the liposomes to particular tissues or cells displaying the human type beta PDGF receptor. Liposomes include emulsions, foams, micelles, insoluble monolayers, liquid crystals, phospholipid dispersions, lamellar layers and the like. In these preparations the immunoglobulin polypeptide or fragment to be delivered is incorporated as part of the liposome, alone or in conjunction with a molecule which is, for example, toxic to the target cells. A liposone suspension containing an immunoglobulin polypeptide can be administered intravenously, locally, topically, etc. in a dose which varies according to, inter alia, the manner of administration, the peptide being delivered, and the stage of disease being treated.

For solid compositions of the anti-PDGF receptor immunoglobulin polypeptides of the invention, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more anti-PDGF receptor inmunoglobulin polypeptides, and more preferably at a concentration of 25%-75%.

For diagnostic purposes, the anti-PDGF receptor immunoglobulin polypeptides may either be labeled or unlabeled. A label is a substance that provides a detectable signal by any of a variety of techniques well known and reported in the art. The immuriaglobulin polypeptides of the invention themselves may be directly labeled. Alternatively, unlabeled antibodies included in the invention may be used in combination with other antibodies (second antibodies) that are labelled and that recognize the anti-PDGF receptor immunoglobulin polypeptides of the present invention. For example, labelled antibodies specific for the constant regions of the anti-PDGF receptor immunoglobulin polypeptides may be used to detect the immunoglobulin polypeptide bound to a sample.

A wide variety of labels may be employed, such as radionuclides, fluors, enzymes, enzyme substrates, enzyme cofactors, enzyme inhibitors, ligands (particularly haptens), etc. Numerous types of immunoassays are available and are well known to those skilled in the art.

The anti-PDGF receptor immunoglobulin polypeptides, and fragments thereof, of the present invention may be used in various immunoassays for detecting PDGF receptor in physiological specimens. Such immunoassay methods may include liquid phase immunoassays and Western blot analysis, competitive and noncompetitive protein binding assays, enzyme-linked immunosorbant assays (ELISA), an others commonly used and widely described in scientific and patent literature, and many employed commercially.

such immunoglobulins and peptides may likewise be employed in immunohistochemical staining techniques by methods well known in the art.

The following example is offered by way of illustration and not limitation.

### EXAMPLE

### Materials

PDGF BB was purchased from Amgen. Anti-phosphotyrosine monoclonal antibody (MAb) PY20 was purchased from ICI. DMEM, RPMI 1640, F12, calf serum, penicillin-streptomycin solution, G418-Neomycin, 200 mM glutamine, 1M HEPES, sodium pyruvate (11 mg/ml), and phosphate buffered saline (PBS) were from GIBCO. Fetal calf serum (FCS) and the monoclonal antibody subtyping kit was from Hyclone. Tris, sodium phosphate, sodium borate, acetic acid, sodium pyrophosphate, sodium fluoride, dithiothreitol (DTT) , ethylenediamirie tetraacetic acid (EDTA), EGTA, sodium dodecyl sulphate (SDS), sodium orthovanadate, sodium chloride (NaCl citric acid, phenyl methyl sulphonyl fluoride (PMSF), bovine serum albumin (BSA), Triton X100, Tween 20, 2,2' Azino-bis(3-ethylbenzthiazoline)-6-sulphonic acid (ABTS), and hydrogen peroxide were from Sigma. Goat anti-mouse peroxidase and hypoxanthine-thiamine (HT) were from Boehringer Mannheim. Gelatin, unstained protein molecular weight markers and nitrocellulose were from BioRad. Prestained high molecular weight protein markers were from BKL. Protein A sepharose CL4B, Protein G sepharose CL4B, Immunopure- binding buffer, F(abl)₂ and Fab preparation kits-were from Pierce. Prepacked PD10 columns were from Pharmacia. ¹²⁵I-Protein A and ¹⁴C-molecular weight markers were from Amersham.
¹²⁵I-diiodo-Bolton-Hunter reagent was from New England Nuclear. Methanol was from Burdick-Jackson. Tissue culture supplies were from Costar. AG01523B cells were obtained from ATCC. HR5 cells were kindly provided by J.A, Escobedo (UCSF). Primary baboon brachial artery smooth muscle cells ware generously provided by J. Anderson and S. Hanson (Emory Univ.).

### Methods

Cell culture. HIH 3T3 cells were routinely maintained in DMEM containing 10% fetal calf serum, 1X penicillin-streptomycin, 2 mM glutamine, and sodium pyruvate (0.11 mg/ml). CHO cells expressing the extracellular domain (pΔ1-5) or the full-length PDGF beta receptor (HR5) were cultured in RPMI containing 10% FCS, 1X penicillin-streptomycin, 2 nM glutamine, sodium pyruvate (0. 11 mg/ml) and G41B (200 µg/ml). Human foreskin fibroblast cells (AGO1523B) were cultured in DMEM containing 10% FCS, lx penicillin-streptomycin, 2 mM glutamine, and sodium pyruvate (0.11 mg/ml). Monoclonal hybridoma cells were maintained in 50:50 DME:RPMI containing 20% FCS, 1X penicillin-streptomycin, 2 mM glutamine, sodium pyruvate (0.1 mg/ml), 1X HT, and 10% macrophage-conditioned medium.

Construction of Truncated Human PDGF beta receptor-expressing cell lines. Truncation of the human PDGF beta receptor cDNA was performed by oligonucleotide-directed deletion mutagenesis. Oligonucleotide-directed in vitro mutagenesis was performed according to a modified method of Kunkel et al. (1987) Meth. Enzymol. 154: 367-382, which is incorporated herein by reference). Initially a 3.9 kb EcoRI-HindIII cDNA fragment of the entire coding region of the human PDGF beta receptor (residues -32 to 499) was subcloned into the ECORI and HindIII sites of m13np1B generating vector mp1BPR (Maniatis, T. et al. (1982) Molecular Cloning: A Laboratory Manual. Cold Spring Harbor Laboratory, Cold Spring Harbor, NY, which is incorporated herein by reference).

Oligonucleotides were designed to delete the portions of the human PDGF beta receptor CDNA that code for amino acids 499-1074 (PRΔ1; GTG TGA GGA ACG GGA AAT TCA TCG AAG GAC ATC CCC CGAC), or amino acids 384-1074 (PRΔ2; GGA AGG TCG ATG TCT AGT TAA TCG AAG GAC ATC CCC CGAC) (Fig 1.). A stop codon was introduced after residue 499 (FRΔ1) or residue 384 (PRΔ2). Verification of subcloning was performed by restriction enzyme digestion analysis and dideoxy chain termination sequencing (Sanger, F. et al. (1977) Proc. Natl. Acad. Sci. USA. 74: 5463-5467, which is incorporated herein by reference).

The modified PDGF beta receptor polypeptides were subcloned into the EcoRI and HindIII sites of the expression vector PBJ1 (Lin, A. et al. (1990) Science 249: 677-679. which is incorporated herein by reference) and cotransfected with vector pSV2Neo (Southern; P.J. and Berg, P. (1982) J. Mol. Appl. Gen. 1: 327-341, which is incorporated herein by reference) at a ratio of 1:10 into CHO-K1 cells by the method of lipofectin reagent uptake (Felgner, P.L. et al. (1987) Proc. Natl. Acad. Sci. USA 84: 7413-7417, which is incorporated herein by reference). Transfected cells were selected for G41B-neomycin resistance, and individual clones were isolated and screened at equal cell density for high level expression of extracellular PDGF beta receptor in serum-free medium. Expression of the modified extracellular PDGF beta receptor proteins was determined by Western blot analysis using a rabbit polyclonal serum (Ab 1-3-5, see Fig 1) raised against a synthetic peptide based on human PDGF receptor residues 51-68 (SVLTLTNLTGLDTGEYFC SEQ ID NO:3). Recombinant clones pΔ1-5 (expressing full-length extracellular PDGF receptor) and pΔ2-7 (expressing domains 1-4 of the extracellular PDGF receptor) were used for subsequent protein production.

Preparation of MAbs against human PDGF beta receptor. Antibodies were developed as described by Harlow and Lane (1988, In Antibodies: A Laboratory Manual, Cold Spring Harbor Laboratory, Cold Spring Harbor, NY, which is incorporated herein by reference). Mice were immunised with partially purified extracellular domain of PDGF beta receptor (pΔ1-5) using 50-100 µg/immunisation). The titre of antibody in the immunised mice was determined using an enzyme-linked immunosorbent assay (ELISA), as follows. 96-well Immunolon II microtitre plates were coated overnight at 4°C with partially purified (10-15%) extracellular domain of PDGF beta receptor (200-300 ng/well). The remaining manipulations were conducted at room temperature. The wells were blocked with 0.05 M Tris, pH 7 containing 100 mM NaCl and 0.5% gelatin for 1 hour. Plates were incubated for 2 hours with various dilutions of mouse sera, washed 5X with wash buffer (0.05 M Tris, pH7, 100 mM NaCl and 0.3% gelatin) and incubated with goat anti-mouse peroxidase (1:1000 in wash buffer) for 1 hour. Plates were washed as previously described and developed with 2,2'-Azino-bis(3 ethylbenzthiazoline)6-sulphonic acid (ABTS, 1 mg/ml) in 0.1 M citric acid, 0.1 M dibasic sodium phosphate, pH 4, containing hydrogen peroxide (0.003% final concentration). The absorbence at 650 nm was determined, and the values were compared to the values obtained with protein purified to a similar extent from conditioned media from CHO cells transfected with the pBJ vector alone.

Mice showing high reactivity were sacrificed and the spleens were isolated. Splenocytes were removed and fused with myeloma cells P3X as described (Harlow and Lane, op. cit.). Hybridomas were screened using the same ELISA, and positive hybridomas were cloned and rescreened- Positive monoclonal cells were cultured and ascites was prepared in Balb/c mice as described (Harlow and Lane, op. cit.). Tissue culture media was used to sub-type the MAbs, using the subtyping kit from HyClone as instructed by the manufacturer.

Purification of Antibodies. Antibodies were purified on Protein A sepharose CL4B as follows. Ascites fluid was diluted 1:5 in Immunopure binding buffer (Pierce) and chromatographed over a Protein A sepharose CL4B column equilibrated in the same buffer. The flow through was collected and the column was washed with Immunopure binding buffer (10 column volumes). The bound IgGs were eluted with 0.1 M glycine, pH 2.8, and collected in tubes containing 2 M Tris, pH 11 (40 µl/ml) as neutralising agent. The peak protein fractions were detected by measuring the absorbence at 280 nm, pooled and dialysed in FBS (2 changes of 4 L each).

Preparation of MAb 2A1E2 F(ab')₂ and Fab Proteolytic Fragments. F(ab')₂ fragments of Mab 2A1E2 were prepared from intact MAb using the immunapure F(ab')₂ preparation kit (Pierce) according to the manufacturer's instructions. Monoclonal antibody 2A1E2 was incubated with immobilised pepsin for 4 hours at 37°C at pH 4.2, and the uncleaved IgG and Fc fragments were separated from the F(ab')₂ fragments using Protein A sepharose CL4B. Fab fragments were similarly prepared using the Pierce Immunopure Fab preparation kit. The IgG was incubated with immobilised papain for 5 hours at 37°C at pH 7, and undigested IgG and Fc fragment. were removed using Protein A. The samples were analysed by SDS-PAGE after enzymatic digestion, and the PDGF beta receptor ELISA was used to determine loss, if any, of antigen recognition.

Immunoprecipitation Assay. Immunoprecipitations were done by a modification of the procedure of Kessler, S.W. (1981, Meth. Enzymol. 73: 442-471, which is incorporated herein by reference). When purified pA1-5 or pA2-7 were used, the protein was incubated with MAb for either 2 hours at room temp or 12 hours at 4°C in immunoprecipitation (IP) buffer (40 mM Tris, pH 8, 100 nM Nacl, 10 mM EDTA, 1 mM EGTA and 1t Triton X100). If full-length receptor was immunoprecipitated, then cells expressing the PDGF beta receptor were solubilised in IP buffer containing 1 µM sodium orthovanadate and 1 mM PMSF, and incubated with the MAb for B-12 hours at 4°C. Then the samples were incubated with a 50% slurry of a 1:1 mixture of Protein A-Sepharose CL4B and Protein G-sepharose CL4B (50-100 µl/sample). After 1-2 hours at 4°C, the resin was washed by 3 cycles of centrifugation and resuspension in IP buffer, and finally the resin was boiled in Laeamli sample solubilising buffer (50-100 µl/sample) (1970, Nature 227: 680-685, which is incorporated herein by reference). The samples were subjected to SDS-PAGE on a 7% or 10% Laemmli gel, and then transferred to nitrocellulose. The western blot was blocked in blocking buffer (0.05 M Tris, pH 8 containing 0.5% NaCl and 4% BSA), and incubated with the primary antibody for 12 hours at 4°c. The nitrocellulose was washed with blocking buffer and incubated with ¹²⁵I-Protein A (0.4 mCi/ml) for 1-2 hours at room temp and exposed to X-ray film.

Radioiodination of PDGF BB. PDGF BB was iodinated by a modification of the Bolton-Hunter procedure described by Duan et al. (1991, J. Biol. Chem. 266: 413-418, which is incorporated herein by reference). Briefly, ¹²⁵I-diiodo Bolton-Hunter reagent (1 mCi) was dried under nitrogen. Then, PDGF BB (2.5 µg) was added to the ¹²⁵I-diiodo Belton-Hunter reagent in 10 µl of 0.1 M sodium borate (pH 8.5), for 15 min at 4°C. The reaction mixture was quenched with 500 µl of 0.1 M sodium borate, 0.2 M glycine, pH 8.5, for 10 min at 4°C. This material was subjected to gel filtration chromatography on a PD10 column previously equilibrated with 0.3 M acetic acid containing 1 mg/ml B5A. Peak radiolabelled protein fractions were detected using a gamma counter, Typically the specific activity of iodinated PDGF BB was 50000 counts/ng.

Binding of ¹²⁵I-PDGF BB to intact HR5 cells. HR5 cells were harvested with PBS containing 2 mM EDTA for 20 min at 37°C. Washed HR5 cells (1x10⁶ cells/100 µl) were incubated in triplicate in suspension with various concentrations of MAb (or F(ab')₂ or Fab fragments) in PBS containing 0.5% BSA for 30 min at room temp. HR5 cells were incubated with ¹²⁵I-PDGF BB (approx. 1 ng/tube) in the absence (total binding) or presence (non-specific binding) of 100-fold excess unlabelled PDGF BB and carrier protein (platelet poor plasma, 50 µl) for 45 **min** at room temp. The final volume of the incubation was 500 µl. The incubation mixture (400 µl) was layered on Ficoll-paque (700 µl) and centrifuged. The supernatant was removed and the radioactivity in the cell pellet was determined.

Phosphorylation Assay. HR5 cells were grown to confluence in 6-well dishes and primary cells were cultured in 100 mm dishes. Cells were washed twice with cold serum-free DMEM, and incubated on ice for 10 min. Cells were preincubated in duplicate with MAb 2A1E2 for 30-45 min on ice on a rotary shaker, and then ligand PDGF BB was added to the wells and the incubation was continued for 1.5-2 hours. Cells were washed twice with cold PBS and solubilised in either Lysis Buffer (100 mM Tris, pH8, 30 mM sodium pyrophosphate, 50 mM sodium fluoride, 5 mM EDTA, 5 mM EGTA, 1% SDS, 100 mM DTT) , or in IP buffer, both containing 1 mM PMSF and 1 µM sodium orthovanadate. Samples were then processed further prior to electrophoresis.

Mitogenesis in human foreskin fibroblast AG01523B cells and baboon smooth muscle cells. Human foreskin fibroblast AG01523B cells and baboon smooth muscle cells were gown to confluence in 96-well dishes. Baboon smooth muscle cells were quiesced by incubating overnight with DMEM containing 0.5% calf serum. Cells were then incubated in triplicate with various concentrations of MAb 2A1E2 in the presence of PDGF BB for 19 hours at 37°C, followed by 5 hours at 37°C with 2 µCi/well of ³H-thymidine. control baboon primary smooth muscle cells were incubated in parallel with a non-specific MAb and control AG01523B cells were incubated with a non-inhibitory anti-PDGF beta receptor Mab (4C5C8). Wells were then washed with ice-cold 5% TCA (2 x 250 µl), and solubilized with 0.25 N NaOH (2 x 100 µl). The solubilized samples were transferred to scintillation vials and radioactivity was determined.

Dimerisation Assay. Confluent HR5 cells were cultured as described above in 100 mm dishes. Cells were washed twice in cold PBS, and incubated with various concentrations of MAb 2A1E2 in PBS containing BSA (1.5 mg/ml) and 25 mM Hepes for 1 hour at 4°C. PDGF BB was added to the cells and the incubation was continued for 2 hours at 4°C. Cells were washed twice in cold PBS, and incubated for 30 min at 4°C with cross-linker B5³ (0.75 mg/plate) in PBS containing 25 mM Hepes. The reaction was terminated by dilution in quench buffer (0.025 M Tris, pH 7.4, containing 150 mM NaCl; 10 ml/plate). Cells were extracted for 20 min at 4°C in IP buffer containing 1mM PMSF and 100 µM sodium orthovanadate [0.5 ml/plate). Cell lysates were immunoprecipitated overnight at 4°C using polyclonal antihuman beta receptor Ab (ABBB, 1:500 dilution). Then, Protein A CL4B (60 µl of a 50% slurry) was added to each sample. After 1 hour at 4°C, beads were washed serially with PBS containing 0.5% NP40, 0,5 M lithium chloride containing 0.5% MP40, 0.5 M lithium chloride, and finally with water. Samples were solubilised with Laemmli sample solubilising buffer, and subjected to SDS-polyacrylamide gel electrophoresis on a 3%-8% gradient gel, followed by Western transfer to nitrocellulose. Western blots were either incubated with antiphosphotyrosine MAb (1:1000), or with Ab88 (1:500 dilution) overnight at 4°C, followed by ¹²⁵I-protein A (0.15 µCi/ml) for 2 hours at room temp. Western blots were then exposed to X-ray film.

### Results

Properties of MAb 2A1E2. MAb 2A1E2 is an IgG₁ monoclonal antibody. Western analysis shows that MAb 2A1E2 recognises non-reduced human PDGF beta receptor (Fig 2, Panel A, Lane 4) but does not recognise reduced protein (Fig 2, Panel A, lane 1). MAb 2AlE2 immunoprecipitates full-length extracellular receptor (pΔ1-5; residues 1-499) from solution (Fig 2, Panel C, Lane 3). However, when purified pΔ2-7 (without domain 5; residues 1-384) is the antigen, there is no reactivity (Fig 2, Panel B, Lane 2). This data indicates that the epitope recognised by MAb 2A1E2 lies within the fifth Ig-like domain, that is, between residues 385-499 of the human PDGF beta receptor.

Dose-dependent inhibition of ¹²⁵I-PDGF BB binding to HR5 cells by MAb 2AlE2. When HR5 cells (CHO-K cells which express the full length human PDGF beta receptor) are incubated first with MAb 2A1E2 followed by ¹²⁵I-PDGF BB, significant (48.1%) inhibition is observed at a concentration as low as 0.1 nM MAb, compared to cells not treated with MAb 2A1E2. When 1 nM or greater concentrations of MAb 2A1E2 are used, there is 100% inhibition of ligand binding to the full-length PDGF beta receptor on the cells (Fig 3, Panel A). When 200 nM of a different, non-inhibitory MAb (4C5C8) to PDGF beta receptor is used in the preincubation, the amount of inhibition is only 36.1%. This is comparable to the effect of 200 nM of a non-relevant MAb (anti-IIb/IIIa) (19.18% inhibition). The amount of ¹²⁵I-PDGF BB binding in the presence of 1 nM MAb 2A1E2 is equivalent to the amount of ligand binding seen in the presence of 1500-fold excess of unlabelled PDGF BB (Fig. 3, Panel A), or the amount of binding of ligand to non-transtected CHO cells that do not express a human PDGF receptor (data not shown).

To determine if the inhibition seen with MAb 2A1E2 was due to steric hindrance, we prepared F(ab')₂ and Fab fragments of the MAb. These proteolytic fragments of the antibody still recognised the PDGF beta receptor in ELISA (data not shown), though their activity was diminished slightly. When these were used in the radiolabelled ligand binding assay, we found that the antibody fragments still inhibited binding of ¹²⁵I-PDGF BB to the full-length PDGF beta receptor on HR5 cells in a concentration-dependent manner (Fig 3, Panel B). However, complete inhibition was seen with 10 nM MAb 2A1E2. F(ab')₂ or Fab fragments, whereas 1 nM intact antibody completely inhibited binding. Binding of ligand in the presence of 1 nM F(ab')₂ fragments was inhibited by 64.5%, and binding in the presence of 1 nM Fab fragments was inhibited by 50%.

Inhibition of Phosphorylation by MAb 2A1E2. As shown in Fig 4, MAb 2A1E2 specifically inhibited PDGF induced phosphorylation in HR5 cells in a concentration-dependent manner, with approximately 50% inhibition occurring at a concentration of 1.3 nM (Lane 4) and 100% inhibition occurring at 13.3 nM MAb 2A1E2 (Lane 5). control nonrelevant MAb (anti-IIb/IIIa) had no effect (Lane 3) and MAb 4C5CB, which was also developed against the human PDGF beta receptor but recognises a different epitope, had no effect on ligand-induced phosphorylation (Lane 8).

Effect of MAb 2A1E2 on PDGF BB Induced Dimerisation of the Human PDGF beta Receptor. Treatment of HR5 cells with PDGF BB results in ligand-mediated phosphorylation (Fig 5, 180 KDa protein in lanes 1-6) and dimerisation (Fig 5, 390 KDa protein in lanes 2 and 6) of the PDGF beta receptor. When HR5 cells are first preincubated with MAb 2A1E2 and then with PDGF BB, dimerisation was inhibited at all the tested concentrations (Fig 5, Lanes 3, 4 and 5). This data indicates that binding of MAb 2A1E2 on the receptor excludes ligand binding and receptor, dimerisation.

Inhibition of Mitogenesis by MAb 2A1E2. As shown in Fig 6, MAb 2A1E2 inhibits PDGF BB-induced mitogenesis in human foreskin fibroblast AGO1523B cells in a concentration-dependent manner, with maximum inhibition (69.55%) occurring at a concentration of 1.3 µM. When a non-inhibitory MAb, 4C5CB, was used, we did not detect significant inhibition of mitogenesis. Increasing the concentration of MAb 2A1E2 did not improve the degree of inhibition, primarily because these cells also express PDGF alpha receptor (data not shown) which is not bound or inhibited by 2A1E2.

We also determined the effect of various concentrations of MAb 2A1E2 on primary smooth muscle cells from baboon artery. PDGF BB-mediated PDGF receptor phosporylation in baboon artery smooth muscle cells was inhibited by 200 nM and 20 nM MAb 2A1E2 (Fig. 7, Lanes 4 and 5, respectively).

As seen in Fig 8, 1 nM MAb 2A1E2 inhibits ³H-thymidine incorporation in the presence of 1-2 ng/ml of PDGF BE by 90%, and 25 nM MAb inhibits mitogenesie by 80% at ligand concentrations ranging from 1-10 ng/ml. Concentrations of MAb 2A1E2 geater than 250 nM inhibit mitogenesis by 90% at all tested concentrations of ligand. When non-relevant MAbs are used there is no significant effect on mitogenesis in baboon smooth muscle cells.

In summary, we have disclosed a monoclonal antibody, MAb 2A1E2, that is highly specific for the human PDGF beta receptor. MAb 2A1E2 inhibits the binding of PDGF to the human type beta PDGF receptor at nanomolar concentrations, and thus inhibits receptor activation as indicated by inhibition of ligand-mediated phosphorylation and dimerisation. The antibody inhibits mitogenesis in vitro at micromolar concentrations. The proteolytic fragments of the MAb retain inhibitory function, as measured by the inhibition of ¹²⁵I-PDGF BB binding (Fig. 3A). MAb 2A1E2 appears to recognise an epitope in the fifth Ig-like domain of the PDGF beta receptor (Fig 2), and binding to this domain appears to prevent dimerisation of the receptor (Fig 5). There is a specific and significant inhibition of ligand-induced autophosphorylation of the PDGF beta receptor (Fig 4), at concentrations as low as 1.3 nM MAb 2A1E2. Consequently, we have found complete inhibition of PDGF induced mitogenesis in HR5 cells (data not shown) at 0,1 µM MAb 2A1E2, and in human foreskin fibroblast cells (AGO1523B) 70% inhibition is achieved at 1 µM MAb 2A1E2 concentration.

MAb 2A1E2 was also tested for cross-reactivity with smooth muscle cells from baboon brachial artery. PDGF BB-induced phosphorylation (Fig 7) and mitogenesis (Fig 8) was inhibited up to 80% by 20-25 nM MAb 2A1E2. Monoclonal antibody 2A1E2 does not cross-react with PDGF receptors from dog, rat, mouse, or pig, and also does not cress-react with human type alpha PDGF receptor (data not shown).

It is of particular interest that, while we have demonstrated that MAb 2A1E2 binds to the fifth Ig-like domain of the PDGF type beta receptor and thus inhibits PDGF-mediated activity, PDGF binds to the third Ig-like domain.

It is to be understood that the above description is intended to be illustrative and not restrictive. Many embodiments will be apparent to those of skill in the art upon reviewing the above description. The scope of the invention should, therefore, be determined not with reference to the above description, but should instead be determined with reference to the appended claims, along with the full scope of equivalents to which such claims are entitled.

## Claims

1. An isolated immunoglobulin polypeptide or an antigen binding fragment thereof that specifically binds to an extracellular domain of the human PDGF-beta receptor.

2. The isolated immunoglobulin polypeptide or antigen binding fragment thereof of claim 1 wherein specific binding of the polypeptide or fragment to the human PDGF-beta receptor has one or more of the following effects:
(a) inhibition of vascular proliferative phenomena and fibrosis;
(b) inhibition of vascular narrowing in vein grafts;
(c) inhibition of vascular narrowing due to accelerated smooth muscle cell migration and proliferation in transplanted organs;
(d) inhibition of non-vascular fibrotic processes; and
(e) inhibition of restenosis.

3. The isolated immunoglobulin polypeptide or antigen binding fragment thereof of claim 1 wherein specific binding of the polypeptide or fragment to the human PDGF-beta receptor has one or more of the following effects:
(a) inhibition of PDGF BB binding to the PDGF-beta receptor;
(b) inhibition of PDGF-induced phosphorylation of PDGF-beta receptor;
(c) inhibition of PDGF-induced dimeritation of PDGF-beta receptors;
(d) inhibition of PDGF-induced mitogenesis of cells expressing PDGF-beta receptor; and
(e) inhibition of PDGF-induced chemotaxis of cells expressing PDGF-beta receptor.

4. The isolated immunoglobulin polypeptide or antigen binding fragment of any one of claims 1 to 3 wherein said immunoglobulin polypeptide or antigen binding fragment is labeled.

5. The isolated immunoglobulin polypeptide or antigen binding fragment of claim 4 wherein said label is cytotoxic.

6. The isolated immunoglobulin polypeptide or antigen binding fragment of any one of claims 1 to 3 wherein said immunoglobulin polypeptide or antigen binding fragment is bi-specific.

7. The isolated immunoglobulin polypeptide or antigen binding fragment of any one of claims 1 to 3 wherein the immunoglobulin polypeptide or antigen binding fragment is an isotype selected from the group consisting of IgG, IgM, IgA or IgD.

8. The isolated immunoglobulin polypeptide or antigen binding fragment of claim 7 wherein the isotype is IgG.

9. The isolated immunoglobulin polypeptide of claim 1 wherein the polypeptide is a monoclonal antibody.

10. The isolated immunoglobulin polypeptide of claim 9 wherein the monoclonal antibody is antibody 2A1E2.

11. The immunoglobulin polypeptide of any one of claims 1 to 3 wherein the polypeptide is chimeric.

12. The isolated immunoglobulin polypeptide or antigen binding fragment of any one of claims 1 to 3 wherein said immunoglobulin polypeptide or antigen binding fragment is humanized.

13. The isolated immunoglobulin polypeptide or antigen binding fragment of any one of claims 1 to 3 wherein said immunoglobulin polypeptide or antigen binding fragment further comprises a non-human complementary determining region and a human framework region.

14. The isolated immunoglobulin polypeptide or antigen binding fragment of claim 13 further comprising the replacement of residues in said framework region that affect the structure of said complementary determining region wherein said immunoglobulin polypeptide or antigen binding fragment possesses antigen recognition.

15. The isolated immunoglobulin or antigen binding fragment of claim 3(a) wherein said inhibition of binding by PDGF BB to the PDGF-beta receptor occurs *in vitro* in Chinese hamster ovary (CHO) cells that express the PDGF-beta receptor with the immunoglobulin polypeptide or antigen binding fragment present at a concentration of about 0.1 nanomolar followed by about 2.0 nanograms per microliter of PDGF BB.

16. The isolated immunoglobulin or antigen binding fragment of claim 3(b) wherein said inhibition of PDGF-induced phosphorylation of EDGE-beta receptor occurs in *vitro* in Chinese hamster ovary (CHO) cells that express the PDGF-beta receptor with the immunoglobulin polypeptide or antigen binding fragment present at a concentration of about 0.13 nanomolar followed by about 100 nanograms per milliliter of PDGF BB.

17. The isolated immunoglobulin or antigen binding fragment of claim 3(c) wherein said inhibition of PDGF-induced dimerization of PDGF-beta receptors occurs *in* vitro in CHO cells that express the PDGF-beta receptor with the immunoglobulin polypeptide or antigen binding fragment present at a concentration of about 13 nanomolar followed by about 100 nanograms per milliliter of PDGF BB.

18. The isolated immunoglobulin or antigen binding fragment of claim 2(d) wherein said inhibition of PDGF-induced mitogenesis occurs in *vitro* in human foreskin fibroblast cells with the immunoglobulin polypeptide or antigen binding fragment present at a concentration of about 0.2 micromolar in the presence of about 50 nanograms per milliliter of PDGF BB.

19. An isolated nucleic acid sequence encoding for the immunoglobulin polypeptide or antigen binding fragment thereof of any one of claims 1 to 18.

20. The isolated nucleic acid of claim 18 wherein the nucleic acid is operably linked to a promoter.

21. The isolated nucleic acid of claim 20 wherein the promoter and the nucleic acid are contained in an expression vector.

22. A cell line transformed with a vector containing a nucleic acid of any of claims 19 to 21.

23. A method of producing an immunoglobulin polypeptide or an antigen binding fragment thereof comprising:
(a) growing a cell line of claim 22 under conditions in which the immunoglobulin polypeptide or antigen binding fragment is expressed; and
(b) isolating the expressed immunoglobulin polypeptide or fragment.

24. The method of claim 23 wherein the cell line is a hybridoma.

25. The method of claim 24, wherein the hybridoma is HB10938.

26. An immunoglobulin polypeptide produced by the method of claim 23.

27. An antigen binding fragment produced by the method of claim 23.
